# EUROPEAN PATENT SPECIFICATION

(11) **EP 2 037 842 B1**
(45) Date of publication and mention of the grant of the patent: **26.02.2014**
(21) Application number: 07804009.4
(22) Date of filing: 02.07.2007
(51) Int. Cl.: A61F 2/07

(54) **ENDOVASCULAR DEVICE AND METHOD OF FORMING SAME**
ENDOVASKULÄRE VORRICHTUNG UND HERSTELLUNGSVERFAHREN DAFÜR
DISPOSITIF ENDOVASCULAIRE ET SON PROCÉDÉ DE FORMATION

(30) Priority: 12.07.2006 GB 0613816
(43) Date of publication of application: 25.03.2009
(73) Proprietor: VASCUTEK LIMITED, Glasgow Renfrewshire PA4 9RR (GB)
(72) Inventor: ASHTON, Timothy, Rawden, WestKilbride KA23 9HL (GB)
(74) Representative: Ede, Eric
(86) International application number: PCT/GB2007/002461
(87) International publication number: WO 2008/007048

(56) References cited:
- WO-A-03/079929
- US-A- 5 990 379

## Description

The present invention relates to a method for forming an endovascular device, such as a stented endovascular device, useful for supporting and repairing portions of a blood vessel.

Aortic aneurysm is characterised by the dilation or ballooning out of part of the wall of the aorta, the artery through which blood flows from the heart to the body. The majority of aortic aneurysms cause little or no symptoms, and small aneurysms can be controlled with beta blockers to reduce blood pressure. However, larger aneurysms pose significant danger to the patient, as rupture of an aneurysm is frequently fatal. An aneurysm may be caused by genetic conditions, e.g. Marfan syndrome, or by other diseases, and forms when the wall of the artery becomes weakened, often due to the build up of plaque (arteriosclerosis). High blood pressure can also increase the likelihood of aneurysm development. Rupture of an aortic aneurysm gives only a 20% chance of survival, so there is a significant emphasis placed on early diagnosis and treatment. In most cases the abdominal aorta, supplying blood to the abdomen, the pelvis, and the legs, is affected (abdominal aorta aneurysm; AAA), but the thoracic aorta can also be susceptible to aneurysm (thoracic aorta aneurysm; TAA).

Conventional methods for treatment of weakened portions of the vasculature are either surgical replacement of the affected portion of the aorta or a more conservative, minimal invasive endovascular repair.

In surgical intervention, the affected part of the blood vessel is replaced with a prosthetic graft. This invasive approach is normally performed under general anaesthesia, so that the patient's abdomen or thorax can be opened and the prosthesis sutured in place of the aneurysmal vessel. Consequently, the method requires the time of a skilled surgeon and prolonged recovery periods for the patient in hospital. Prosthetic grafts normally used for such replacement are typically made from polyester fabric, which may be woven or knitted, and may be sealed with a sealant, for example gelatine or collagen. One such graft is shown in European Patent application published under No. EP 0183365 (Maini et al.).

More recently endovascular repair techniques have been developed in which an endovascular device is introduced using a catheter. When correctly placed, the stent of the endovascular device is expanded and the catheter removed. Since the catheter can be introduced via the femoral artery, the technique requires only minimal invasive procedures and consequently the patient enjoys a faster recovery. The procedure was first described by Parodi (Annals of Vascular Surgery vol 5, pages 491-499, 1991 and US 5,578,072), and the technique is rapidly replacing conventional surgery as the preferred method of treating AAA and TAA. Many different types of devices useful for endovascular repair are now available, for example a resiliently engaging endovascular element described in US 6,635,080 (Vascutek) or a tubular fabric liner having a radially expandable supporting frame and a radiopaque marker element stitched to the liner as disclosed in US 6,203,568 (Medtronic).

Although there are a number of different grafts designed to date, all grafts share the limitation of complex construction based on time consuming manual assembly. In particular, the stent elements are generally attached to the graft material by hand sewing. The use of hand sewing is not only time-consuming and therefore costly, but also leads to variations in consistency giving rise to quality control issues. Efforts to avoid the variations in quality of the endovascular devices have included the development of specialised sewing machines designed to overcome the difficulties of sewing through the walls of long tubes at a significant distance from the end. Examples include a sewing apparatus as described by Rawson (WO 2004/072348), which is capable of stitching through the walls of tubes, or a machine as described by Shonteff (US 6,295,940) which pinches the wall of a fabric tube to allow stitches to be placed. An alternative approach is adopted by Philips (GB 2,349,827) where an embroidery machine is used to attach nitinol wire to a flat sheet of textile. The sheet is then formed into a tube by sewing its edges together. However each of these methods still has the disadvantage of requiring a large number of stitches to be formed to produce the graft.

Previous approaches to reduce the complexity of the process include sandwiching the stent element between two layers of fabric. Hunt (WO 2005/096998) describes a stent element of a basket or lattice type of structure, and which has the inner and outer layers of fabric joined through openings in this structure. Lentz (US 5,843,166) describes securing individual stent elements to a covering by encapsulating them between two layers allowing relative motion between the stent and the cover, by forming pockets between the two layers.

WO 03/079929 discloses the features of the preamble of claim 1.

None of the prior art documents discussed above teaches the forming of an endovascular device with two graft layers and a stent element located therebetween, the graft layers being attached by laser welding in such a way that movement of the stent element with respect to the graft layers is prevented.

The present invention provides an endovascular device comprising a first tubular graft layer having a stent element located therearound, said device further including a second graft layer at least part of which covers a portion of the stent element, wherein said stent element includes at least one discrete ring and wherein said first and second tubular graft layers are attached together thereby preventing relative movement of the stent element and each graft layer.

In one embodiment the first and second graft layers are attached together in such a way to prevent relative movement of at least one (preferably each) stent element with respect to the graft layers.

In one embodiment the first and the second graft layers are transparent to light. Preferably the first and the second graft layers are transparent to near infrared light, for example to light having a wavelength between 400 and 1500 nm or, more particularly, between 600 and 1300 nm.

In one embodiment the first and/or the second graft layers are made from polyester, polypropylene or polyethylene, particularly a high tenacity material such as Dyneema^{®} (a polyethylene).

Optionally the first and/or second graft layers can be formed using a composite yarn having a core of material with a high melting point and a sheath of material with a lower melting point. Such a composite yarn ensures a good attachment by welding without loss of tensile strength, since the core will be substantially unaffected by the welding process. A suitable material for the core is polyester. Suitable materials for the sheath include polypropylene or polyethylene. The composite yarn can be formed by known techniques, such as co-extrusion.

In one embodiment the second graft layer is tubular. The second graft layer is a strip placed longitudinally over at least one stent element, preferably over each stent element. The embodiment preferably comprises at least two, and more preferably four or more stent elements. The strip construction of the second graft layer can be advantageous in reducing the bulkiness of the endovascular device to assist delivery via small diameter catheters. Generally the second graft layer can be formed from multiple strips (for example 2, 3, 4, 5 or 6 strips) each placed longitudinally with respect to the main axis of the first graft layer and each of which is placed over one or more stent elements, preferably over each stent element.

In one embodiment the second graft layer is formed from monofilament polyester.

In another embodiment the second graft layer is formed from monofilament polypropylene.

In one embodiment the first and the second graft layers are attached to each other at multiple discrete attachment sites, thereby securing the stent element(s) and preventing relative movement of the stent elements with respect to each of the graft layers.

In one embodiment, the endovascular device will have multiple stent elements, for example up to 40 stent elements. In one embodiment there can be 4 to 30, 10 to 25, for example 15 to 25 stent elements.

In one embodiment, the attachment sites between the first and the second tubular graft layers are formed by laser welding.

WO 02/083798 describes laser welding using a non-aqueous dye able to absorb laser light of a near-infrared wavelength. The dye converts the energy from the laser into heat without significant energy loss via fluorescence. WO 02/083798 refers to the dye being applied to a thermoplastic material for industrial applications. Whilst other welding techniques or heat bonding could be used, such techniques tend to fuse the full thickness of the fabric at the melt point. Dye-mediated laser welding however only melts the fabric at the interfacial surface.

In one embodiment of the present invention a near-infrared absorbing dye is applied to either the outer surface of the first graft layer or to the inner surface of the second graft layer, or to both layers. Application of the dye can conveniently be performed by spraying or dipping, although other techniques can also be used - for example the dye can be applied by brush or felt pad, or by ink-jet printing.

Optionally, the dye can be applied to the inner surface of a tubular graft (eg. to the second graft layer when in tubular form) by turning the graft inside out to aid application.

In one embodiment the dye is selected from the group consisting of a tris (dialkylaminophenyl) aminium dye coupled with one anion, a nickel dithiolate, a nickel dithiolene, a tetrakis (dialkylaminophenyl) aminium dye coupled with one anion, a cyanine, a squarylium, a tetrakis (dialkylaminophenyl) diimonium dye coupled with two anions, and a croconium.

In a further aspect the present invention provides a dye as described above for use in the manufacture of an endovascular device comprising a thermoplastic material. Suitable thermoplastic materials are described above.

The laser-welded attachment site(s) can be at discrete pre-determined locations on the device. Thus, in one embodiment, the dye can be applied at discrete pre-determined locations on the device or the dye can be applied to a broad area of the endovascular device, e.g. to an area of between 0.1mm² and 10mm². In some embodiments the dye could even coat substantially the complete surface of the first and/or second graft member.

In another embodiment, the stent element is made of nitinol wire, laser cut nitinol or stainless steel.

In one embodiment, each discrete ring of the stent element is made of a multiplicity of sub-rings formed by winding nitinol wire. In one embodiment, one or each discrete ring of the stent element is formed in an undulating shape. In alternative embodiments, other shapes of ring elements can be used, if required.

In one embodiment, some or each of the ring elements are arranged to be substantially parallel with the other ring elements.

In one embodiment, the second graft layer has a thickness of from 0.05mm to 0.5mm.

In one embodiment the second graft layer is a tubular structure and the first tubular graft layer is coaxially arranged within the lumen of the second graft layer, creating an annular gap therebetween. In this embodiment the first graft layer has an outer diameter ranging from 8 to 40mm and the inner diameter of the second tubular layer greater than the outer diameter of the first layer from 0.1 to 1.0mm. From 2 to 40 stent elements (for example 8 to 25 stent elements) are disposed within the annular gap between the first and the second graft layers, spaced equidistant from each other along the length of the device and are fixed in position via laser-welded attachment sites between the two graft layers in such a manner that the stent elements do not move relative to the graft layers. In an alternative embodiment the second graft layer is formed from at least two and preferably four, strips of material, preferably of polyester monofilament, located longitudinally and attached by laser-welded attachment sites.

The present invention also provides a method of producing an endovascular device, said method comprising locating a stent element comprising at least one discrete ring element coaxially onto a first inner tubular graft layer and locating a second outer graft layer to at least partially cover the stent element and attaching said inner and outer graft layers securely together, thereby preventing relative movement of the stent element and each graft layer.

In one embodiment the inner and the outer graft layers are transparent to light. Preferably the inner and the outer graft layers are transparent to near infrared light, for example to light having a wavelength ranging from 400nm to 1500nm, for example, ranging from 600nm to 1300nm.

In one embodiment the inner and/or the outer graft layers are made from polyester, polypropylene or polyethylene.

In one embodiment the outer graft layer is tubular. In an alternative embodiment the outer graft layer is a strip placed longitudinally over at least one stent element, preferably over each stent element.

In one embodiment, the endovascular device will have multiple stent elements, for example up to 40 stent elements. In one embodiment the device can have 4 to 30 stent elements, for example 10 to 25, or 15 to 25 stent elements.

The strip construction of the outer graft layer can be advantageous in reducing the bulkiness of the endovascular device to assist delivery via small diameter catheters. Generally the outer graft layer can be formed from multiple strips (for example 2, 3, 4, 5 or 6 strips) each placed longitudinally with respect to the axis of the inner graft layer, and each of which is placed over one or more stent elements, preferably over each stent element.

In one embodiment the outer graft layer is formed from monofilament polyester.

In one embodiment the inner and the outer graft layers are attached to each other at multiple discrete attachment sites, thereby securing the stent element(s).

In one embodiment, the attachment sites between the inner and the outer tubular graft layers are formed by laser welding, said method comprising locating a stent element comprising at least one discrete ring element coaxially onto a first inner tubular graft layer, locating a second outer graft layer to at least partially cover the stent element; and attaching said inner and outer graft layers securely together by laser welding thereby preventing relative movement of the stent element and each graft layer.

In one embodiment a near-infrared absorbing dye is applied to either the inner surface of the outer layer or to the outer surface of the inner layer, or to both layers. Application of the dye can conveniently be performed by spraying or dipping, although other techniques can also be used - for example the dye can be applied by brush or felt pad or by ink-jet printing.

Optionally, the dye can be applied to the inner surface of a tubular graft by turning the graft inside out to aid application.

Suitably, the dye can be applied in discrete areas, for example as circular spots of diameter 2 mm to 3 mm.

The laser-welded attachment site(s) can be at discrete pre-determined locations on the device. Thus, in one embodiment, the dye can be applied at discrete pre-determined locations on the device or the dye can be applied to a broad area of the endovascular device, e.g. to an area between 0.1mm² and 10 mm². In some embodiments, the dye could even coat substantially the complete surface of the inner and/or outer graft member. Suitably a diode laser with a maximum power of 150W and wavelength of 940 nm can be used. A suitable power setting is 20 to 40 W. A suitable traverse speed is 1.0 to 2.5m/min.

In one embodiment, the stent element is made of nitinol wire, laser cut nitinol or stainless steel.

In one embodiment, each discrete ring of the stent element is made of a multiplicity of sub-rings formed by winding nitinol wire. In one embodiment, one or each discrete ring of the stent element is formed in an undulating shape. In alternative embodiments, other shapes of ring elements can be used, if required.

In one embodiment, some or each of the ring elements are arranged to be substantially parallel with the other ring elements.

In one embodiment, the outer graft layer has a thickness of from 0.05mm to 0.5mm.
In one embodiment the outer tubular graft layer may be turned inside out for convenient application of the dye to the inner surface.

In one embodiment a jig which is transparent to near-infrared laser light is positioned onto the assembled device before laser welding. Optionally pressure is applied towards the central longitudinal axis to bring the first and the second tubular structures into contact. The area of contact is at least those areas where dye has been applied.

In one embodiment the jig is constructed from polycarbonate or acrylic polymers.

In one embodiment the jig has an inner surface with grooves corresponding to the desired position of a stent element.

In one embodiment the jig is provided in segmented form, e.g. is in the form of two half, releasably attachable cylinders. The half cylinders can be clamped together by any suitable means such as cable ties, hose clips or clamps.

In one embodiment an assembly comprising a mandrel, first and second graft layers, the stent element disposed between the first and the second graft layers and the jig is placed in a fixing means for holding the assembly in place. The dye has already been applied to the areas required prior to assembly of the graft layers and stent elements, and the location of assembly into the jig. In one embodiment the fixing means allows the assembly to be conveniently moved for laser welding. Control of the fixing means is coupled to a near-infrared laser such that the areas having dye applied are irradiated with as much energy as required to create at least one laser-weld. Conveniently a power ranging between 20 to 50 W, and particularly of about 40 W achieves good results.

The present application will be now further described with reference to the following non-limiting examples and figures in which:
Figure 1 shows a stented endovascular device according to the invention.
Figure 2 shows a partial longitudinal cross-section through a length of the stented endovascular device as shown in Fig. 1.
Figure 3 shows an alternative embodiment of a stented endovascular device according to the invention in which the stent elements are secured by longitudinal strips.
Figure 4 shows a longitudinal cross-section through a stented endovascular device of Figs 1 or 3 located in a jig.
Figure 5 shows the graft of Figure 1 and the jig used for its manufacture.

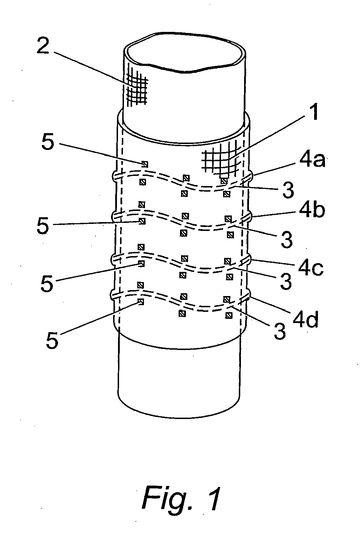

Fig. 1 shows a stented endovascular device comprising an outer graft layer 1 made of polyester fabric or similar, which forms an outer layer of the endovascular device, and an inner tubular graft layer 2, also made of polyester fabric or similar, which forms an inner layer of the endovascular device. The two layers 1, 2 are arranged coaxially and have an annular gap therebetween. A stent element 3 constructed of four non-interconnected ring elements 4a, 4b, 4c, 4d and formed from windings of nitinol wire or similar, is positioned within the annular gap located between the outer and the inner graft layers 1, 2. The stent element 3 is fixed in position via attachment sites 5 between the outer graft layer 1 and the inner graft layer 2. As illustrated each ring element 4 is formed in an undulating shape, and is arranged to be substantially parallel with the other ring elements 4. Other shapes of ring elements can be used, if required.

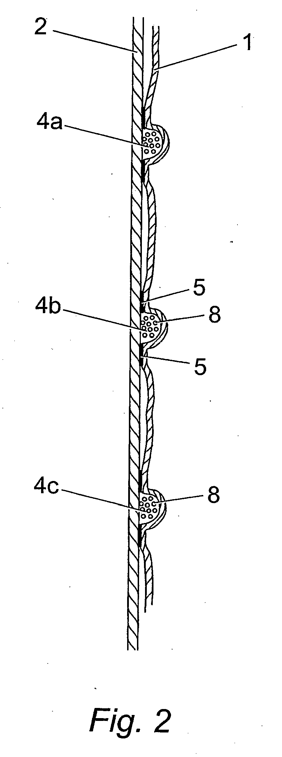

Attachment sites 5 are shown in more detail in Fig. 2. Only a portion of the stent element 3 is illustrated. Fig. 2 illustrates that each ring element 4a, 4b, 4c is made of a multiplicity of sub-rings 8, formed by winding nitinol wire. Each ring element 4 is fixed in place separately via the attachment sites 5 located above and below the ring element 4. At these attachment sites 5, the adjacent surfaces of the inner graft layer 1 and the outer graft layer 2 are interconnected via laser welding, thereby preventing relative movement of the stent elements 4 with respect to the graft layers 1, 2.

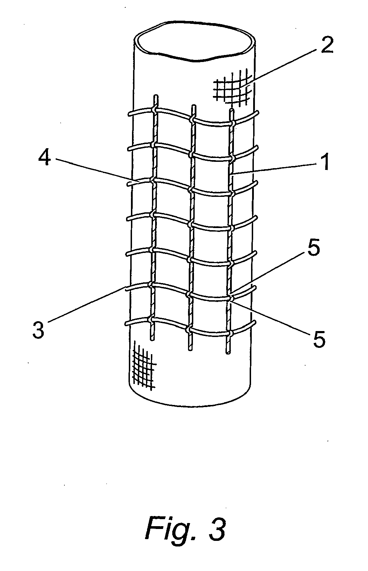

Figure 3 shows an alternative embodiment of the stented endovascular device according to the invention, wherein the stent element 4 is disposed on the outer surface of the inner graft layer 2 as described for Figure 1 and is fixed in place by the outer graft layer 1, which is in form of fabric strips, partially covering the inner graft layer 2. The stent element is formed from seven ring elements 4 located between the outer and inner graft layers 1, 2.

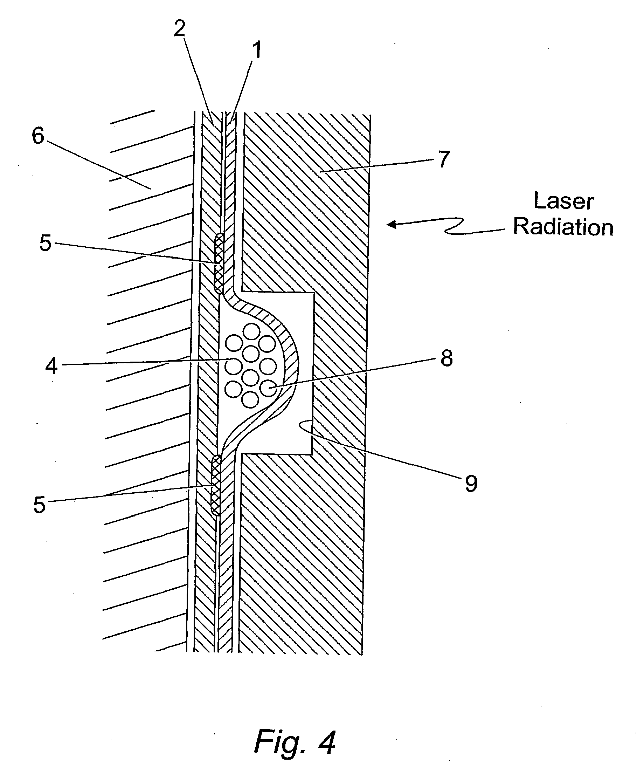

Figure 4 shows a longitudinal cross-section through the wall of a stented endovascular device of Figs. 1 or 3, with a jig 7 located in position for the welding step. The inner graft layer 2 is placed onto a mandrel 6 and the stent element comprising ring elements 4 is disposed on the outer surface of the inner graft layer 2. Ring element 4 is formed from multiple windings 8 of nitinol wire. A dye is applied to discrete portions of the inner surface of the second graft layer 1, which is then placed over at least a portion of the stent element to form the outer layer of the endovascular device.

Pressure is applied inwardly to the surface of the second graft layer 1 to ensure a good contact between the outer graft layer 1 and the inner graft layer 2. The pressure can conveniently be applied via jig 7, and the inner graft layer 2 is welded by exposure to laser radiation to the second graft layer 1 at points 5 either side of each ring element 4 to prevent its movement relative to the graft layers 1, 2.

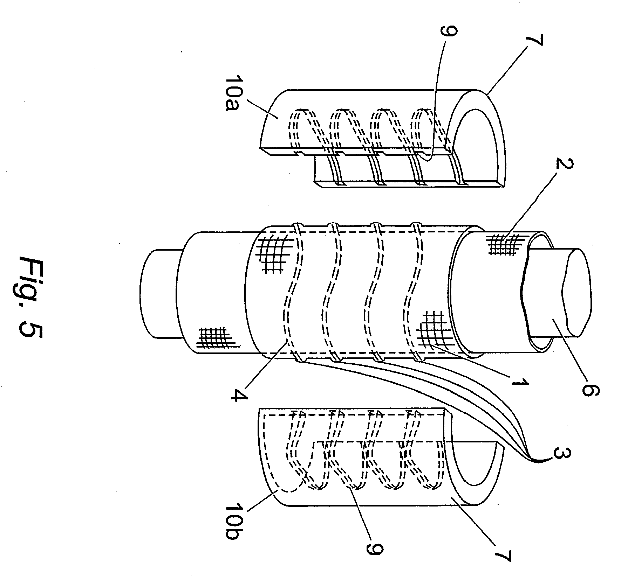

Figure 5 shows a partial exploded view of the endovascular device and jig. The jig 7 is illustrated as formed in two half cylinders 10a, 10b. On the inner surface of each half cylinder 10a, 10b is shown grooves 9 which correspond to and locate over each ring element 4, forming a close fit therewith. The jig 7 is formed from transparent material, such as acrylic. The two half cylinders 10a, 10b are located over the assembled device and clamped into position prior to the photo welding step.

### Example 1

A stented endovascular device as illustrated in Fig. 1 was manufactured by locating a polyester tubular graft layer of 0.12mm thickness onto a 14mm diameter mandrel such that it fits snugly and without wrinkles. Eighteen pre-formed ring elements formed from multiple windings of nitinol wire are then placed on the fabric in approximately the desired positions. A second layer of tubular polyester graft material of 0.1 mm thickness was treated on its inner surface with absorbent dye (LD120B from Gentex) before being located over the ring stents. Alternatively dye is applied at the sites of each of the 18 rings. The adjacent surfaces of the two graft layers are then attached via laser welding though exposure to laser light of near infra-red wavelength at a traverse speed of 2.5m/min. A diode laser with a maximum power of 150W and a wavelength of 940nm was used at a power setting of 30W and the spot size was adjusted to 2-3mm.

### Example 2

Optionally the method as described in Example 1 may further include placing a jig onto the assembled device prior to laser welding. The jig has an inner surface with grooves corresponding to the intended position of the stent elements for correctly positioning the latter and holding the stent elements in place. The jig is transparent to light of a wavelength which is absorbable by the dye. Irradiation with laser light of the appropriate wavelength results in energy absorption by the dye and local heating of the adjacent sides of the two graft layers at the areas having dye applied and held in contact with each other. The two tubular graft layers are welded together, thereby fixing the stent elements in position.

### Example 3.

Optionally the method of Examples 1 or 2 can have the outer layer of polyester fabric replaced by polypropylene material or polyethylene material. The lower melting point of the polypropylene or polyethylene polymer avoids the potential for damage to the polyester substrate. A diode laser with a wavelength of 940nm was needed at a power setting of 15W.

A further option is to use a fabric constructed of composite yarns. For example, it is possible to use a co-extruded yarn in which a core of polyester is surrounded by a sheath of polypropylene or polyethylene. The outer sheath melts at a lower temperature than the core and so ensures good attachment without loss of tensile strength.

This process may be viewed as fusion bonding rather than true welding, as only one of the joined materials is melted.

## Claims

1. An endovascular device comprising a first inner tubular graft layer (2) having a stent element (3) located therearound, said device further including a second outer graft layer (1) at least part of which covers a portion of the stent element (3), wherein said stent element (3) includes at least one discrete ring, **characterised in that** said second outer graft layer (1) is formed from at least one strip placed over said stent element (3) and placed longitudinally with respect to the inner graft layer (2) and wherein said first and second graft layers (2, 1) are attached together thereby preventing relative movement of the stent element (3) and each graft layer.

2. The endovascular device of Claim 1 wherein the inner and the outer graft layers (2, 1) are transparent to near infrared light having a wavelength ranging from 400nm to 1500nm, preferably from 600nm to 1300nm.

3. The endovascular device of either one of Claims 1 and 2 wherein the inner and/or the outer graft layers (2, 1) are made from polyester, polypropylene or polyethylene.

4. The endovascular device of any one of Claims 1 to 3 wherein the outer graft layer (1) has a thickness of from 0.05mm to 0.5mm.

5. The endovascular device of any one of Claims 1 to 4 wherein the outer graft layer (1) is formed from 2 to 6 strips placed longitudinally with respect to the inner graft layer (2).

6. The endovascular device of any one of Claims 1 to 5 wherein the endovascular device has multiple stent elements (3), preferably from 4 to 30 stent elements (3).

7. The endovascular device of Claims 1 to 6 wherein the outer graft layer (1) is located over each stent element (3).

8. The endovascular device of any one of Claims 1 to 7 wherein the inner and outer graft layers (2, 1) are attached to each other at multiple discrete attachment sites (5) formed by a laser welding step thereby securing the stent elements (3).

9. The endovascular device of Claim 8 wherein the laser welding step uses a non-aqueous dye able to absorb laser light of a near infra-red wavelength.

10. The endovascular device of Claim 9 wherein the dye is selected from the group consisting of a tris (dialkylaminophenyl) aminium dye coupled with one anion, a nickel dithiolate, a nickel dithiolene, a tetrakis (dialkylaminophenyl) aminium dye coupled with one anion, a cyanine, a squarylium, a tetrakis (dialkylaminophenyl) diimonium dye coupled with two anions, and a croconium.

11. A method of forming the endovascular device of any one of Claims 1 to 10, said method comprising
i. locating a stent element (3) comprising at least one discrete ring element coaxially onto a first inner tubular graft layer (2);
ii. locating a second outer graft layer (1) formed from at least one strip to at least partially cover the stent element (3); and
iii. attaching said inner and outer graft layers (2, 1) securely together by laser welding, thereby preventing relative movement of the stent element (3) and each graft layer (2, 1).

12. The method of forming the endovascular device as claimed in Claim 11 wherein the laser welding step is performed by
i. locating the first tubular graft layer (2) onto a mandrel (6);
ii. placing the stent element (3) onto said first tubular graft layer (2);
iii. applying a near-infrared absorbing dye to the inner surface of the second outer graft layer (1) or to the outer surface of the first inner tubular graft layer (2), or to both layers;
iv. bringing the first and the second graft layers (2, 1) into contact so that the area of contact is at least those areas where dye has been applied; and
v. irradiating the areas where dye has been applied with a near-infrared laser with as much energy as required to create at least one laser-weld.

13. The method of Claim 12 further comprising the step of positioning a jig (7) which is transparent to near-infrared laser light onto the endovascular device before laser welding.

## Patentansprüche

1. Eine endovaskuläre Vorrichtung, beinhaltend eine erste innere röhrenförmige Transplantatschicht (2) mit einem Stent-Element (3), das darum angeordnet ist, wobei die Vorrichtung ferner eine zweite äußere Transplantatschicht (1) umfasst, wobei mindestens ein Teil davon einen Abschnitt des Stent-Elements (3) abdeckt, wobei das Stent-Element (3) mindestens einen separaten Ring umfasst, **dadurch gekennzeichnet, dass** die zweite äußere Transplantatschicht (1) aus mindestens einem Streifen gebildet ist, der über dem Stent-Element (3) platziert ist und bezüglich der inneren Transplantatschicht (2) in Längsrichtung platziert ist, und wobei die erste und die zweite Transplantatschicht (2, 1) aneinander befestigt sind, wodurch eine Relativbewegung des Stent-Elements (3) und jeder Transplantatschicht verhindert wird.

2. Endovaskuläre Vorrichtung gemäß Anspruch 1, wobei die innere und die äußere Transplantatschicht (2, 1) für Nah-Infrarotlicht durchlässig sind, das eine Wellenlänge im Bereich von 400 nm bis 1500 nm, vorzugsweise von 600 nm bis 1300 nm aufweist.

3. Endovaskuläre Vorrichtung gemäß Anspruch 1 oder 2, wobei die innere und/oder die äußere Transplantatschicht (2, 1) aus Polyester, Polypropylen oder Polyethylen hergestellt ist/sind.

4. Endovaskuläre Vorrichtung gemäß einem der Ansprüche 1 bis 3, wobei die äußere Transplantatschicht (1) eine Dicke von 0,05 mm bis 0,5 mm aufweist.

5. Endovaskuläre Vorrichtung gemäß einem der Ansprüche 1 bis 4, wobei die äußere Transplantatschicht (1) aus 2 bis 6 Streifen gebildet ist, die bezüglich der inneren Transplantatschicht (2) in Längsrichtung platziert sind.

6. Endovaskuläre Vorrichtung gemäß einem der Ansprüche 1 bis 5, wobei die endovaskuläre Vorrichtung mehrere Stent-Elemente (3), vorzugsweise von 4 bis 30 Stent-Elemente (3) aufweist.

7. Endovaskuläre Vorrichtung gemäß den Ansprüchen 1 bis 6, wobei die äußere Transplantatschicht (1) über jedem Stent-Element (3) angeordnet ist.

8. Endovaskuläre Vorrichtung gemäß einem der Ansprüche 1 bis 7, wobei die innere und die äußere Transplantatschicht (2, 1) an mehreren separaten Befestigungsstellen (5), die durch einen Laserschweißschritt gebildet werden, aneinander befestigt sind, wodurch die Stent-Elemente (3) fixiert werden.

9. Endovaskuläre Vorrichtung gemäß Anspruch 8, wobei der Laserschweißschritt einen nichtwässrigen Farbstoff verwendet, der Laserlicht mit einer Nah-Infrarotwellenlänge absorbieren kann.

10. Endovaskuläre Vorrichtung gemäß Anspruch 9, wobei der Farbstoff ausgewählt ist aus der Gruppe, bestehend aus Tris(dialkylaminophenyl)aminium-Farbstoff, gekoppelt mit einem Anion, einem Nickeldithiolat, einem Nickeldithiolen, einem Tetrakis(dialkylaminophenyl)aminium-Farbstoff, gekoppelt mit einem Anion, einem Cyanin, einem Squarylium, einem Tetrakis(dialkylaminophenyl)diimonium-Farbstoff, gekoppelt mit zwei Anionen, und einem Croconium.

11. Ein Verfahren zum Bilden der endovaskulären Vorrichtung gemäß einem der Ansprüche 1 bis 10, wobei das Verfahren Folgendes beinhaltet:
i. Anordnen eines Stent-Elements (3), beinhaltend mindestens ein separates Ringelement, koaxial auf einer ersten inneren röhrenförmigen Transplantatschicht (2);
ii. Anordnen einer zweiten äußeren Transplantatschicht (1), gebildet aus mindestens einem Streifen, so dass sie das Stent-Element (3) mindestens teilweise abdeckt; und
iii. Befestigen der inneren und der äußeren Transplantatschicht (2, 1) fest aneinander durch Laserschweißen, wodurch eine Relativbewegung des Stent-Elements (3) und jeder Transplantatschicht (2, 1) verhindert wird.

12. Verfahren zum Bilden der endovaskulären Vorrichtung gemäß Anspruch 11, wobei der Laserschweißschritt durchgeführt wird durch:
i. Anordnen der ersten röhrenförmigen Transplantatschicht (2) auf einer Spindel (6);
ii. Platzieren des Stent-Elements (3) auf der ersten röhrenförmigen Transplantatschicht (2);
iii. Auftragen eines Nah-Infrarot absorbierenden Farbstoffs auf die innere Oberfläche der zweiten äußeren Transplantatschicht (1) oder auf die äußere Oberfläche der ersten inneren röhrenförmigen Transplantatschicht (2) oder auf beide Schichten;
iv. In-Kontakt-Bringen der ersten und der zweiten Transplantatschicht (2, 1), so dass der Kontaktbereich mindestens denjenigen Bereichen entspricht, auf die Farbstoff aufgetragen wurde; und
v. Bestrahlen der Bereiche, auf die Farbstoff aufgetragen wurde, mit einem Nah-Infrarotlaser mit so viel Energie wie erforderlich, um mindestens eine Laserschweißnaht zu erzeugen.

13. Verfahren gemäß Anspruch 12, ferner beinhaltend den Schritt des Positionierens einer Spannvorrichtung (7), die für Nah-Infrarotlaserlicht durchlässig ist, auf der endovaskulären Vorrichtung vor dem Laserschweißen.

## Revendications

1. Un dispositif endovasculaire comprenant une première couche de greffon tubulaire interne (2) présentant un élément d'endoprothèse (3) situé autour de celle-ci, ledit dispositif comportant de surcroît une deuxième couche de greffon externe (1) dont au moins une partie recouvre une portion de l'élément d'endoprothèse (3), dans lequel ledit élément d'endoprothèse (3) comporte au moins un anneau discret, **caractérisé en ce que** ladite deuxième couche de greffon externe (1) est formée à partir d'au moins une bande placée par-dessus ledit élément d'endoprothèse (3) et placée longitudinalement par rapport à la couche de greffon interne (2) et dans lequel lesdites première et deuxième couches de greffon (2, 1) sont attachées ensemble, empêchant de ce fait le déplacement relatif de l'élément d'endoprothèse (3) et de chaque couche de greffon.

2. Le dispositif endovasculaire de la revendication 1 dans lequel les couches de greffon interne et externe (2, 1) sont transparentes à la lumière infrarouge proche ayant une longueur d'onde comprise dans la gamme allant de 400 nm à 1 500 nm, préférablement de 600 nm à 1 300 nm.

3. Le dispositif endovasculaire de l'une ou l'autre des revendications 1 et 2 dans lequel les couches de greffon interne et/ou externe (2, 1) sont réalisées à partir de polyester, polypropylène ou polyéthylène.

4. Le dispositif endovasculaire de l'une quelconque des revendications 1 à 3 dans lequel la couche de greffon externe (1) a une épaisseur allant de 0,05 mm à 0,5 mm.

5. Le dispositif endovasculaire de l'une quelconque des revendications 1 à 4 dans lequel la couche de greffon externe (1) est formée à partir de 2 à 6 bandes placées longitudinalement par rapport à la couche de greffon interne (2).

6. Le dispositif endovasculaire de l'une quelconque des revendications 1 à 5 dans lequel le dispositif endovasculaire a des éléments d'endoprothèse multiples (3), préférablement de 4 à 30 éléments d'endoprothèse (3).

7. Le dispositif endovasculaire des revendications 1 à 6 dans lequel la couche de greffon externe (1) est située par-dessus chaque élément d'endoprothèse (3).

8. Le dispositif endovasculaire de l'une quelconque des revendications 1 à 7 dans lequel les couches de greffon interne et externe (2, 1) sont attachées l'une à l'autre au niveau de sites d'attache discrets multiples (5) formés par une étape de soudage par laser, fixant solidement de ce fait les éléments d'endoprothèse (3).

9. Le dispositif endovasculaire de la revendication 8 dans lequel l'étape de soudage par laser utilise un colorant non aqueux capable d'absorber de la lumière laser d'une longueur d'onde de l'infrarouge proche.

10. Le dispositif endovasculaire de la revendication 9 dans lequel le colorant est sélectionné dans le groupe consistant en un colorant tris (dialkylaminophényl) aminium couplé avec un anion, un dithiolate de nickel, un dithiolène de nickel, un colorant tétrakis (dialkylaminophényl) aminium couplé avec un anion, une cyanine, un squarylium, un colorant tétrakis (dialkylaminophényl) diimonium couplé avec deux anions, et un croconium.

11. Une méthode pour former le dispositif endovasculaire de l'une quelconque des revendications 1 à 10, ladite méthode comprenant :
i. situer un élément d'endoprothèse (3) comprenant au moins un élément en anneau discret de façon coaxiale sur une première couche de greffon tubulaire interne (2) ;
ii. situer une deuxième couche de greffon externe (1) formée à partir d'au moins une bande pour recouvrir partiellement au moins l'élément d'endoprothèse (3) ; et
iii. attacher lesdites couches de greffon interne et externe (2, 1) de façon solide ensemble par soudage par laser, empêchant de ce fait le déplacement relatif de l'élément d'endoprothèse (3) et de chaque couche de greffon (2, 1).

12. La méthode pour former le dispositif endovasculaire tel que revendiqué dans la revendication 11 dans laquelle l'étape de soudage par laser est effectuée en
i. situant la première couche de greffon tubulaire (2) sur un mandrin (6) ;
ii. plaçant l'élément d'endoprothèse (3) sur ladite première couche de greffon tubulaire (2) ;
iii. appliquant un colorant absorbant l'infrarouge proche sur la surface interne de la deuxième couche de greffon externe (1) ou sur la surface externe de la première couche de greffon tubulaire interne (2), ou sur les deux couches ;
iv. amenant les première et deuxième couches de greffon (2, 1) en contact de façon à ce que la zone de contact soit au moins les zones où du colorant a été appliqué ; et
v. irradiant les zones où du colorant a été appliqué avec un laser infrarouge proche avec autant d'énergie qu'il est nécessaire pour créer au moins une soudure laser.

13. La méthode de la revendication 12 comprenant de surcroît l'étape consistant à positionner un bâti (7) qui est transparent à la lumière laser infrarouge proche sur le dispositif endovasculaire avant le soudage par laser.
